(19) Europäisches Patentamt
European Patent Office
Office européen des brevets

(11) Numéro de publication: **0 000 009**
**B1**

(12) # FASCICULE DE BREVET EUROPEEN

(21) Numéro de dépôt: **78400019.2**

(22) Date de dépôt: **14.06.78**

(51) Int. Cl.³: **C 07 D 453/04**

(54) **Procédé d'oxydation de quinine en quininone et quinidinone.**

(30) Priorité: **15.06.77 FR 7718398**

(43) Date de publication de la demande:
**20.12.78 Bulletin 78/01**

(45) Mention de la délivrance du brevet:
**23.07.80 Bulletin 80/15**

(84) Etats contractants désignés:
**BE CH DE FR GB LU NL SE**

(56) Documents cités:
**DE - B - 1 165 604**
**FR - A - 2 332 278**
**NL - A - 7 612 951**

(73) Titulaire: **Devinter S.A.**
**2 Boulevard Royal**
**Luxembourg (LU)**

(72) Inventeur: **Bourrelly, Jacques**
**4 avenue de Chateau**
**F - 92190 Meudon (FR)**

(74) Mandataire: **de Haas, Michel**
**Cabinet Beau de Lomenie 55 rue d'Amsterdam**
**F - 75000 Paris 8ème (FR)**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

Courier Press, Leamington Spa, England.

EP 0 000 009 B1

Procédé d'oxydation de quinine en quininone et quinidinone

La présente invention concerne un procédé d'oxydation de type Oppenauer par un réactif nouveau et son application à la fabrication de quininone et de quinidinone, intermédiaires de synthèse de la quinidine.

Rabe et col. en 1909 [Ann. 364, 346] avaient déjà proposé une préparation de quininone et de quinidinone à partir de quinine ou de quinidine. L'oxydation s'effectuait avec de très faibles rendements.

Woodward [JACS *67*, 1425 (1945)] et Rabe [Ber 51, 446 (1918)] ont réalisé par la suite des synthèses permettant d'isoler un produit avec un bon rendement.

Sueles les études plus récentes par dichroisme circulaire de la structuree des produits isolés a permis de définir que le produit isolé — le plus souvent à caractère huileux — et présentant en solution une mutarotation était en fait un mélange de deux isomères : la quininone et la quinidinone (par analogie avec la structure des deux alcaloïdes naturels du quinquina : la quinine et la quinidine).

On a pu mettre en évidence, en particulier grâce aux travaux de synthèse de USKOKOVIC (USP 3 753 992 — 1973), que la seule forme cristalline, p.f. —98 à 101°C avait la structure de la quinidinone. La quininone ne pouvant être isolée que sous forme d'une huile.

Les structures sont indiquées sur la figure 1.

La quininone et la quinidinone que se différencient par l'isomérie au carbone asymétrique en $C_8$ du noyau quinuclidine sont en général deux formes en équilibre, ce qui explique les phénomènes de mutarotation en solution jusqu'ici constatés.

En présence d'une base de $p_K$ suffisant à énoliser la cétone, l'équilibre entre la quininone et la quinidinone s'opère entre les formes indiquées à la figure 2.

Ce mécanisme explique les différences de rendements obtenus et le manque de définition des caractères physicochimiques du produit communément appelé "quininone" qui n'est en fait que le mélange des deux stéréo-isomères présentant le phénomène de mutarotation en solution.

La plupart du temps, le rendement d'oxydation s'évalue par dosage spectrophotométrique UV à 360 nm.

L'oxydation de Oppenauer s'effectue dans le cas des alcanoïdes du quinquina seulement en présence de cétone de la famille des benzophénones.

Cette cétone, jouant le rôle d'accepteur de protons, doit remplir un certain nombre de conditions strictes en ce qui concerne:

— le potentiel d'oxydation,
— la réactivité à basse température,
— les contraintes d'encombrement stériques,
— l'absence de réactions secondaires.

De nombreux dérivés de benzophénone ont été testés et ont donné des résultats sensiblement équivalents. Les autres cétones aromatiques ou aliphatiques (acétone, cyclohexanone, acétophénone, etc.,) sont sans activité dans le cas des produits qui nous intéressent.

Pour des raisons économiques, seules la benzophénone et la fluorénone sont pratiquement utilisées.

Dans ces conditions, l'oxydation de la quinine et de la quinidine en quininone déjà décrite [WOODWARD, VENDLER, JACS *67*, 1245 (1945)] [WARNHOFF, REYNOLDS, J. Org. Chem. *28*, 1431 (1963)] ne s'effectue qu'en présence de 5 moles équivalents de cétone accepteur. Lorsque des quantités inférieures sont mises en présence, les conditions de l'équilibre OPPENAUER-MEERWEIN-POONDORFF sont réalisées et la quininone formée se trouve en présence de quantités variables d'impuretés, principalement: quinine, quinidine, épiquinine, épiquinidine, quinicine (quinotoxine), ce qui a pour effet de réduire le rendement de la réaction, les sous-produits formés devant être séparés et purifiés avant d'être recyclés (on note en particulier que la présence — même en faibles proportions — d'épibases diminue considérablement les rendements de cristallisation de la quinine et de la quinidine à partir de leurs solutions saturées.

La réaction s'effectuant sous forme énolique, il est nécessaire d'introduire une base de $p_K$ suffisant à énoliser l'ensemble des formes cétoniques concourant à la réaction. Une quantité de 2 à 7 moles équivalents s'est avérée nécessaire dans les diverses expériences décrites dans la littérature. Les bases employées, du fait de leur $p_K$, sont les alcoolates, et principalement les alcoolates de sodium, de potassium et d'aluminium, des alcools méthylique, éthylique, isopropylique et tert-butylique.

Ces produits coûteux, dangereux d'emploi, présentent de plus l'inconvénient de libérer un alcool primaire en quantité importante.

La présence de cet alcool a deux inconvénients principaux:

—entrant en réaction, il provoque des réductions parasites de la quininone formée en sous-produits secondaires gênants (déplacement de l'equilibre vers les bases et épibases);

—il nécessite des traitements de purification coûteux pour le recyclage des solvants (distillation fractionée).

L'objet de l'invention est principalement d'utiliser une base présentant les caractéristiques idéales

de $p_K$ et de compatibilité qui soit un dérivé "in situ" de la cétone utilisée comme accepteur de protons.

En effet, on connaît des anions radicalaires formés par addition d'un atome de métal alcalin (Me) sur une cétone de la série des benzophénones [BENT, HARRISSON, JACS *66*, 969 (1944)].

Il s'agit d'un anion radiculaire présentant une tautomérie entre deux formes principales:

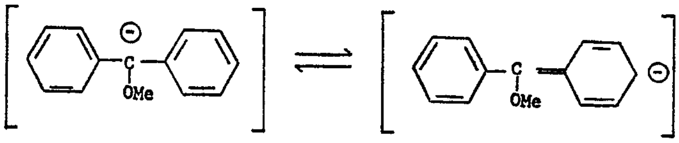

dans le cas de la benzophénone.

Pour la fluorénone, l'équilibre peut se représenter de facon analogue:

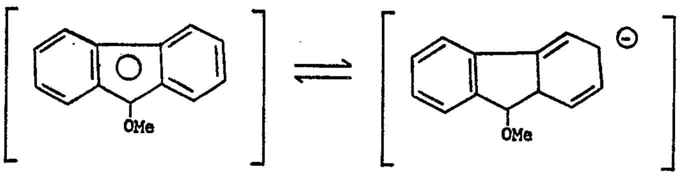

Les "Ketyl" sont préparés par simple addition du métal alcalin à une solution de cétone dans un solvant aromatiques approprié.

On peut également faire réagir un amalgame (par exemple un amalgame de sodium).

On utilisera donc comme oxydant de la quinine un "Kétyl" obtenu par interaction d'un métal alcalin sur une diphénylcétone, plus particulièrement sur la benzophénone ou la fluorénone.

On constate que l'alcaloïde base, mis en présence de la solution de "Kétyl", réagit très rapidement pour donner la forme cétonique désirée avec un rendement quantitatif. On note l'absence de sous-produits et d'impuretés dans le milieu réactionnel.

Le réaction de la quinine et du "Kétyl" est réalisée dans un milieu solvant; ce solvant est choisi parmi les hydrocarbures aromatiques en $C_6$—$C_9$, les hydrocarbures cycliques saturés en $C_6$—$C_9$ ou les hydrocarbures aliphatiques correspondant à des coupes naphta ou gas-oil.

Les avantages de cette invention sont multiples:

— obtention avec un excellent rendement de la cétone désirée,

— simplification de la réaction qui s'opère dans des conditions optimales de durée et de température,

— remplacement d'un alcoolate coûteux et dangereux par l'emploi d'un métal alcalin (principalement de sodium) d'usage répandu dans l'industrie chimique et bon marché,

— élimination des problèmes de recyclage de solvants, car il ne se forme plus une mole d'alcool aliphatique par mole d'alcoolate engagé.

L'ensemble de ces propriétés a permis, comme on le verra dans les exemples décrivant ci-après, à titre non limitatif, les applications de l'invention, d'obtenir dans de bonnes conditions économiques la quininone et la quinidinone à partir de mélanges d'épibases considérées jusqu'ici comme des sous-produits fatals de l'hémisynthèse des alcaloïdes du quinquina (en particulier de la quinidine).

Exemple 1

On met en suspension 2,4 g de sodium dans 40 ml de xylène anhydre au reflux, afin d'obtenir une bonne dispersion.

Après refroidissement à 90°C, on introduit lentement 36 g de benzophénone anhydre.

La solution prend alors la couleur bleu-vert, caractéristique du "Kétyl" (présence d'électrons).

Dans un second réacteur, on dissout à l'ébullition 13 g de quinine base anhydre dans 50 ml de xylène.

On coule cette solution dans la solution précédente. Après 60 minutes au reflux, la réaction est complète.

La solution xylénique est traitée à l'eau (20 ml), puis extraite par 100 ml d'acide sulfurique dilué à 20%.

On neutralise par addition d'ammoniaque la solution sulfurique froide.

Il se sépare une huile qui cristallise lentement après ensemencement.

Les cristaux qui se forment sont composés de quinidinone; comme la quininone et la quinidinone sont en équilibre dans l'huile obtenue, la précipitation de la quinidinone a pour conséquence de déplacer ledit équilibre vers la production de quinidinone.

Poids obtenu: 12,2 g (94% de la théorie) de quinidinone

Chromatographie en couches minces

Eluant acétone 80/DMF 20, 1 tache, Rf: 0,8

Exemple 2

On met en suspension 24 g de sodium dans 40 ml de toluène anhydre, en chauffant, pour obtenir par fusion une fine division du métal.

En parallèle, on dissout 360 g de benzophénone anhydre dans 450 ml de toluène sec. Ces deux solutions sont mélangées et maintenues au reflux pendant 15 minutes.

130 g de quinine base anhydre sont dissous à l'ébullition dans 500 ml de toluène anhydre. Cette solution est versée à chaud dans la solution de benzophénone "Kétyl". Après 30 minutes au reflux, la réaction est complète.

La solution toluénique refroidie à 50°C est lavée à l'eau, puis extraite par 700 ml d'acide sulfurique dilué.

La solution sulfurique froide est neutralisée par addition d'ammoniaque. Une huile décante. Elle est séparée et cristallisée par ensemencement et traitement approprié.

Poids d'huile obtenu: 124 g de quininone, p.f. 102°C
CCM 1 tache, Rf 0,8

Exemple 3

A une solution de fluorénone "Kétyl," préparée à partir de 180 g de fluorénone et de 130 g de sodium dans le xylène anhydre, on ajoute une solution de 65 g de mélange, de composition suivante:

| | |
|---|---|
| Epiquinine | 35% |
| Epiquinidine | 24% |
| Quinine | 7% |
| Quinidine | 3% |
| Quininone | 2% |
| Quinotoxine | 2% |
| Epicinchonine + épicinchonidine | 12% |
| Alcaloïdes divers + autres cinchonines | 15% |

dans le toluène anhydre.

Les solutions mélangées sont maintenues au reflux pendant 5 heures.

La réaction est alors complète.

On obtient après traitement approprié 55 g d'un produit contenant:
quininone + quinidinone: 39 g
cinchoninone + cinchonidinone: 16 g

Exemple 4

On réalise une solution de fluorénone "Kétyl" à partir de 9 g de fluorénone dans le toluène anhydre.

A cette solution, on ajoute à chaud une solution de 37 g de quinidine base anhydre dans 150 ml de toluène anhydre.

Après 15 minutes de reflux, la réaction est complète.

Après traitement approprié, on obtient 36 g de quinidinone pure (CCM) (97% de la théorie).

**Revendications**

1. Procédé d'oxydation de quinine en quininone et quinidinone, au moyen d'une réaction d'oxydation du type Oppenauer, caractérisé en ce que ladite réaction est effectuée à l'aide d'un réactif basique dit "Kétyl" résultant de l'action directe, sur une diphénylcétone, d'un métal alcalin en milieu solvant.

2. Procédé selon la revendication 1, caractérisé en ce que la diphénylcétone est choisie parmi les benzophénones et les fluorénones.

3. Procédé selon l'une des revendications 1 et 2, caractérisé en ce que le solvant utilisé pour la préparation du "Kétyl" est choisi parmi les hydrocarbures aromatiques en $C_6$—$C_9$, les hydrocarbures

cycliques saturés en $C_6$—$C_9$ et les hydrocarbures aliphatiques correspondant à des coupes naphtha et gas-oil.

## Claims

1. A process for the oxidation of quinine to quininone and quinidinone by means of an Oppenauer type oxidation reaction, characterized in that said reaction is carried out with a basic reagent known as "Ketyl" resulting from the direct action, on a diphenylketone, of an alcaline metal in a solvent medium.

2. A process according to claim 1, characterized in that the diphenylketone is selected from benzophenones and fluorenones.

3. A process according to any one of claims 1 and 2, characterized in that the solvent used for the preparation of "Ketyl" is selected from $C_6$—$C_9$ aromatic hydrocarbons, $C_6$—$C_9$ saturated cyclic hydrocarbons and aliphatic hydrocarbons corresponding to naphtha cuttings and gas oil.

## Patentansprüche

1. Verfahren zur Oxydierung von Chinin zu Chininon und Chinidinon mittels einer Oxydierungsreaktion nach Oppenauer, dadurch gekennzeichnet, dass die genannte Reaktion mit Hilfe eines basischen Reagens, des sogenannten "Ketyl" durchgeführt wird, das aus der unmittelbaren Wirkung eines Alkalimetalls in einem Lösungsmittel auf ein Diphenylketon entsteht.

2. Verfahren gemäss Anspruch 1, dadurch gekennzeichnet, dass das Diphenylketon unter Benzophenonen und Fluorenonen gewählt wird.

3. Verfahren gemäss einem der Ansprüche 1 und 2, dadurch gekennzeichnet, dass das für die Herstellung von "Ketyl" verwendete Lösungsmittel unter aromatischen Kohlenwasserstoffen mit 6 bis 9 Kohlenstoffatomen, gesättigten ringförmigen Kohlenwasserstoffen mit 6 bis 9 Kohlenstoffatomen und aliphatischen Kohlenwasserstoffen gewählt wird, die Naphthaschnitten und Gasöl ensprechen.

FIGURE 1

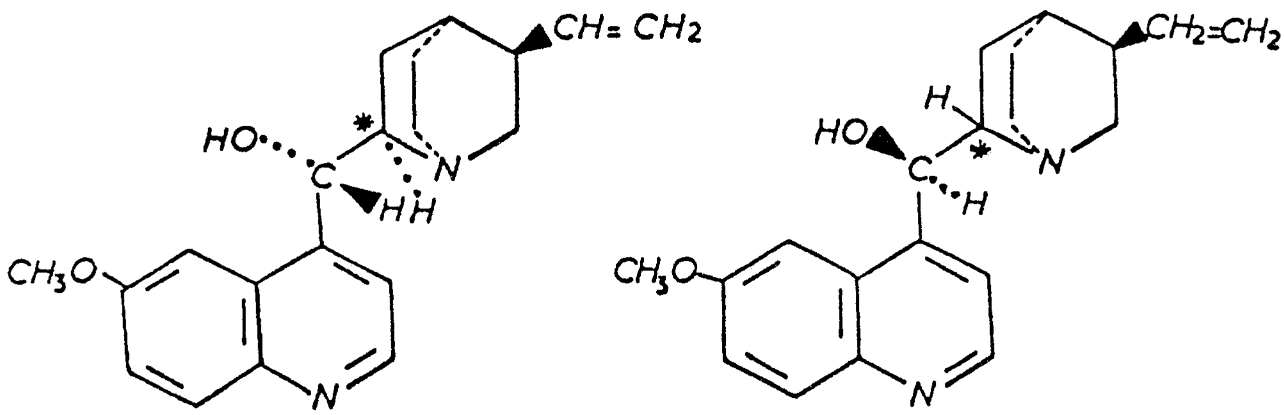

QUINIDINE

QUININE

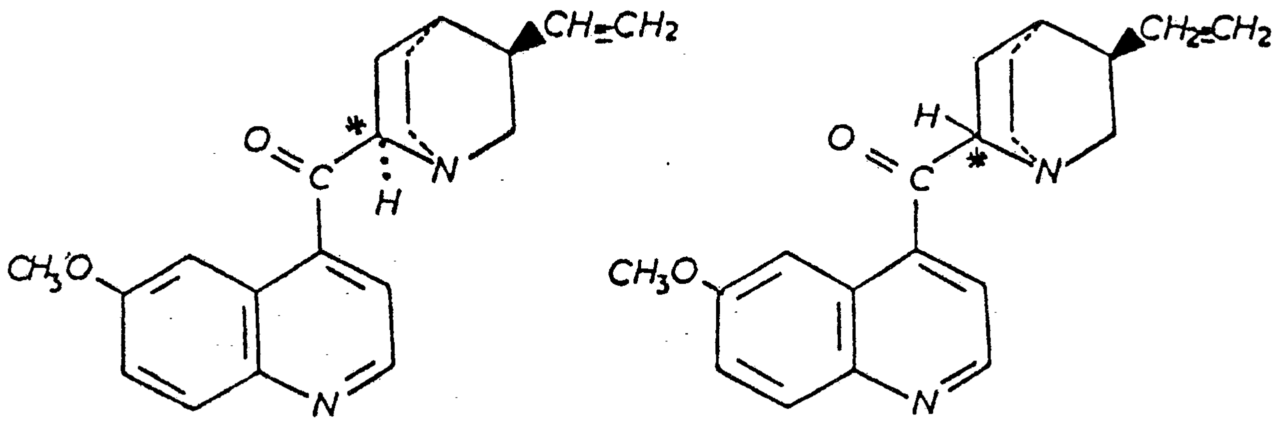

QUINIDINONE

QUININONE

FIGURE 2

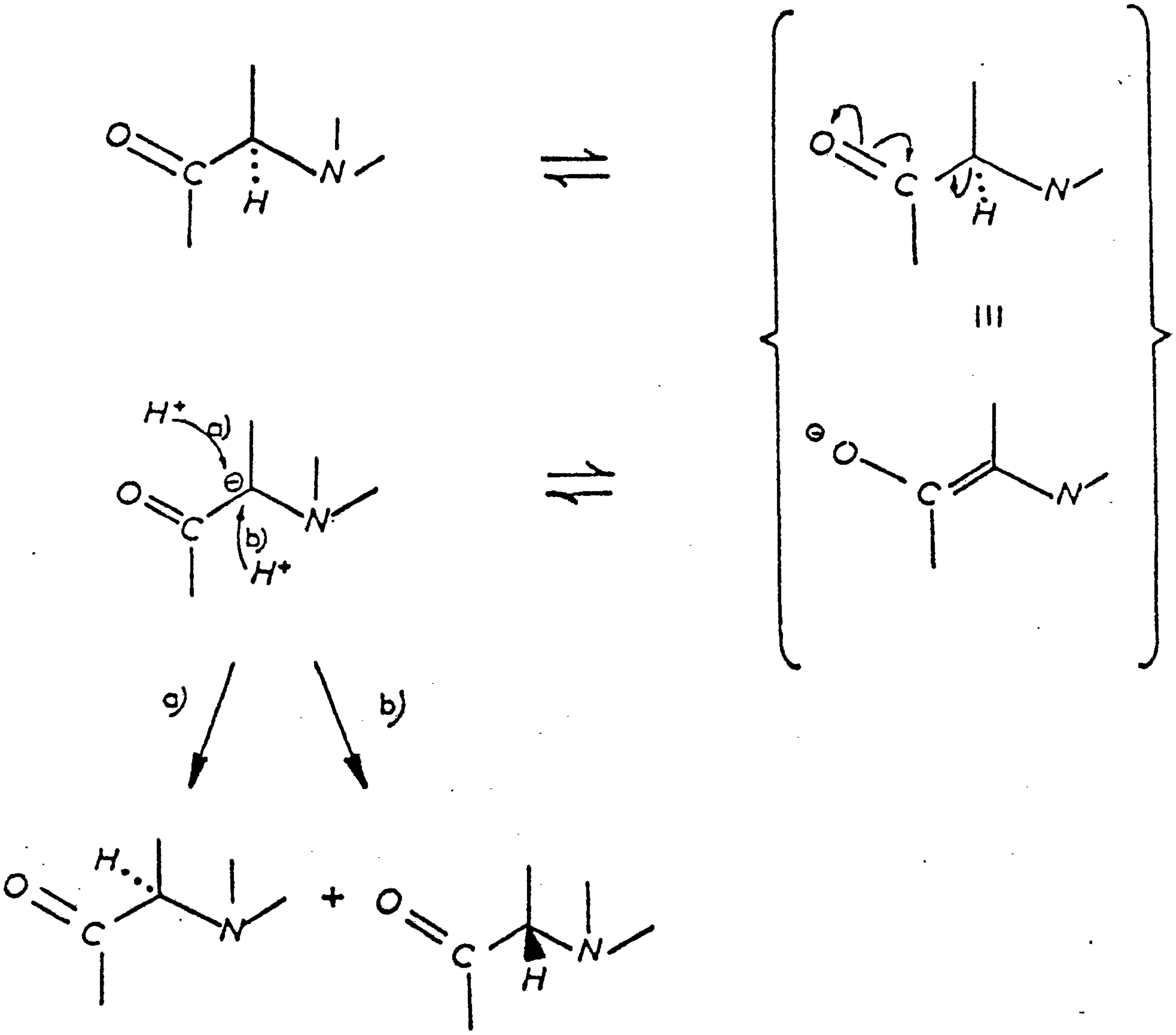


QUINIDINONE + QUININONE